# EUROPEAN PATENT SPECIFICATION

(11) **EP 2 257 337 B1**
(45) Date of publication and mention of the grant of the patent: **10.04.2013**
(21) Application number: 09714993.4
(22) Date of filing: 11.02.2009
(51) Int. Cl.: A61N 1/39, A61M 5/142, A61N 1/02, A61N 1/372, A61N 1/362

(54) **ATRIAL DEFIBRILLATION USING AN IMPLANTABLE DEFIBRILLATION SYSTEM**
ATRIALE DEFIBRILLATION MIT EINEM IMPLANTIERBAREN DEFIBRILLATIONSSYSTEM
DÉFIBRILLATION ATRIALE UTILISANT UN SYSTÈME DE DÉFIBRILLATION IMPLANTABLE

(30) Priority: 27.02.2008 US 64288
(43) Date of publication of application: 08.12.2010
(73) Proprietor: Livnat, Avi, 63574 Tel Aviv (IL); Iddan, Gavriel Joseph, 34602 Haifa (IL); Azar, Lazaro Salomon, 53631 Givatayim (IL)
(72) Inventor: Livnat, Avi, 63574 Tel Aviv (IL); Iddan, Gavriel Joseph, 34602 Haifa (IL); Azar, Lazaro Salomon, 53631 Givatayim (IL)
(74) Representative: McLeish, Nicholas Alistair Maxwell
(86) International application number: PCT/US2009/033786
(87) International publication number: WO 2009/108502

(56) References cited:
- EP-B- 0 764 451

## Description

### FIELD

The present embodiments relate to atrial defibrillation. More specifically, the present embodiments relate to defibrillation of the atria using an implantable defibrillation system.

### BACKGROUND

Atrial fibrillation is a cardiac arrhythmia (abnormal heart rhythm) that involves at least one of the upper chambers of the heart, such as the right atrium or the left atrium. To defibrillate a fibrillating atrium, an electrical pulse is delivered to the heart at a specific moment in the cardiac cycle. Atrial defibrillation, using an implantable atrial defibrillation system, includes automatically detecting atrial fibrillation and automatically delivering the electrical pulse to the upper chambers of the heart. The delivery of the electrical pulse may be a painful procedure for the patient and may hinder the use of automatically activated implantable atrial defibrillators. EP 0 764 451 B1, which discloses all the features of the preamble of claim 1, discloses an implantable heart defibrillator for defibrillation with low-energy pulses.From one perspective, delivering an electrical pulse with an energy that is too high may cause pain for the patient. However, from a different perspective, if the energy is too low, the defibrillation will not be successful. Accordingly, atrial defibrillation that is safe, effective, and/or reduces the discomfort to a patient may be desired.

### SUMMARY

In order to address the need for improved atrial defibrillation, an implantable atrial defibrillator and method for use are disclosed herein. According to one aspect, an implantable heart defibrillator for use with an electrode lead system is provided. The implantable heart defibrillator includes an electrode lead connector that is connectable to the electrode lead system. A sensor is connected to the electrode lead connector. The sensor is operable to sense a condition of a heart and emit a condition signal that identifies the condition. A control unit is connected to the sensor. The control unit is operable to identify whether a state of fibrillation exists from the condition signal and emit a command signal if the state of fibrillation exists. A shock pulse generator is connected to the control unit and the electrode lead connector. The shock pulse generator is operable to emit at least one defibrillation shock to the electrode lead connector upon receipt of the command signal. The at least one defibrillation shock comprises at least one pulse having a voltage of more than 600 volts and a time duration of 30 to 100 microseconds.

In another aspect, a method for defibrillating an atrium with an implantable defibrillation system is provided. The method includes detecting when an atrium in the heart is in a state of fibrillation. At least one electrical pulse parameter is set. The at least one electrical pulse parameter defines an electrical pulse having a defibrillation voltage of at least 600 volts and a pulse duration of 30 - 100 microseconds. A first electrical pulse in accordance with the at least one electrical pulse parameter is generated and discharged to an atrium of the heart using a discharge electrode and a receiving electrode of the implantable defibrillation system. The defibrillation voltage of the first electrical pulse is at least 600 volts and the pulse duration is 30 - 100 microseconds.

In yet another aspect, a system for defibrillation of an atrium of a heart is provided. The system includes a memory and a processor in communication with the memory. The memory includes processor readable instructions that are executable with the processor. The processor readable instructions are executed to receive condition signals from one or more sensors, determine when the atrium of the heart is in a fibrillation state using the cardiac functioning signals, generate a first defibrillation electrical pulse with a field strength of 100 - 700 volts/cm between a discharge electrode and a receiving electrode and a time duration of 30 - 100 microseconds, and discharge the first defibrillation electrical pulse from the discharge electrode and a receiving electrode to defibrillate the atrium of the heart.

### BRIEF DESCRIPTION OF THE DRAWINGS

The present embodiments may be better understood with reference to the following drawings and description. Non-limiting and non-exhaustive embodiments are described with reference to the following drawings. The components in the drawings are not necessarily to scale, emphasis instead being placed upon illustrating the principles of the present embodiments. In the drawings, like referenced numerals designate corresponding parts throughout the different views.

Figure 1 illustrates one embodiment of a implantable defibrillation system;

Figure 2 illustrates one embodiment of a control system;

Figure 3A illustrates an implantable defibrillation system in the subclavian vein, and Figure 3B illustrates an implantable defibrillation system in the right atrium;

Figure 4 illustrates one embodiment of a control unit;

Figure 5 illustrates another embodiment of an implantable defibrillation system;

Figure 6 illustrates one embodiment of an electrode lead system with a bifurcated main lead;

Figures 7A - 7B illustrate exemplary implantable defibrillation systems with bifurcated main leads;

Figure 8 illustrates one embodiment of an electrode lead system with a single electrode on the main lead;

Figures 9A - 9B illustrate implantable defibrillation systems with a single electrode on the main lead;

Figure 10 illustrates one embodiment of an electrode lead system with a plurality of electrodes on the main lead;

Figures 11A - 11C illustrate implantable defibrillation systems with a plurality of electrodes on the main lead;

Figures 12A - 12E illustrate exemplary locations for the discharge electrode and receiving electrode; and

Figure 13 illustrates a flow diagram of a method for performing atrial defibrillation.

### DETAILED DESCRIPTION

The present embodiments relate to defibrillation of an atrium using an implantable defibrillation system. In one embodiment, the pain and/or discomfort of atrial defibrillation may be reduced by defibrillating the heart using an electrical pulse with a field strength of 100 - 700 volts/cm and a time duration of 30 - 50 microseconds. The electrical pulse may have a discharge voltage of at least 600 volts. The short time duration of the electrical pulse is intended to reduce pain during and after the discharge of the electrical pulse to the atrium. Accordingly, the pain and/or discomfort of atrial defibrillation may be reduced by controlling the contraction induced by the electrical shocks to the surrounding muscles. In order to control the contraction induced by the electrical shock, an electrical pulse with a field strength of 100-700 volts/cm across the heart muscle ensures that the electrical pulse provides the energy needed to safely and effectively defibrillate the heart and reduces the contraction to the electrically conducting muscles surrounding the heart muscle. Therefore, it may be advantageous to use an electrical pulse with a field strength of 100-700 volts/cm and a time duration of 30 - 100 microseconds when defibrillating an atrium.

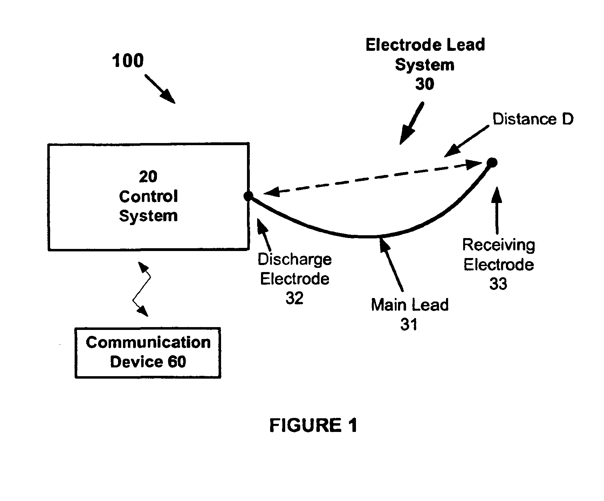

Figure 1 shows an implantable defibrillation system 100. The implantable defibrillation system 100 includes a control system 20 and an electrode lead system 30. The control system 20 is coupled with the electrode lead system 30. Herein, the phrase "coupled with" includes directly connected to or indirectly connected through one or more intermediate components. Such intermediate components may include both hardware and software based components. The electrode lead system 30 is disposed in or around a human heart and includes a discharge electrode 32 and a receiving electrode 33. The control system 20 generates an electrical pulse, which is discharged from the discharge electrode 32 to the receiving electrode 33. The electrical pulse has a field strength of 100-700 volts/cm. To achieve this field strength, the electrical pulse may have a high voltage, for example, greater than 600 volts.

Alternatively, the implantable defibrillation system 100 may include additional, different, or fewer components. For example, the system 100 may include an antenna that is used for receiving or transmitting information to a communication device 60, as shown in Figure 1. As will be discussed below, the antenna may send and receive information, which may be used to communicate with the control system 20. The antenna may be used to deliver information, such as trigger information, warning signals, distance information, voltage difference information, or software updates, to the control system 20. Communication between the control system 20 and communication device 60 may be, as shown in Figure 1, wireless.

The communication between the control system 20 and the communication device 60 may be one-way or bi-directional. The communication may be half or full duplex. Further, the control system 20 may push communication to the communication device 60, or, alternatively or in addition thereto, respond to requests from the communication device 60. The communication may be used to program, set up, and monitor the control system 20, as well as interrogate the control system 20 for alarm data, sanity data, and for offloading from the device stored patient's heart activity and device activity data.

The implantable defibrillation system 100 discussed herein may be implemented in any of a number of configurations, such as an implantable miniature atrial defibrillator, implantable heart defibrillator, defibrillation implant, an implantable cardioverter defibrillator, a pacemaker system, a ventricular defibrillation system, other system used for atrial defibrillation, or any combination thereof. For example, in the illustration above, the implantable miniature atrial defibrillator is an implantable defibrillation system 100. In another example, the implantable defibrillation system 100 may be a combination of a pacemaker system and an atrial defibrillator.

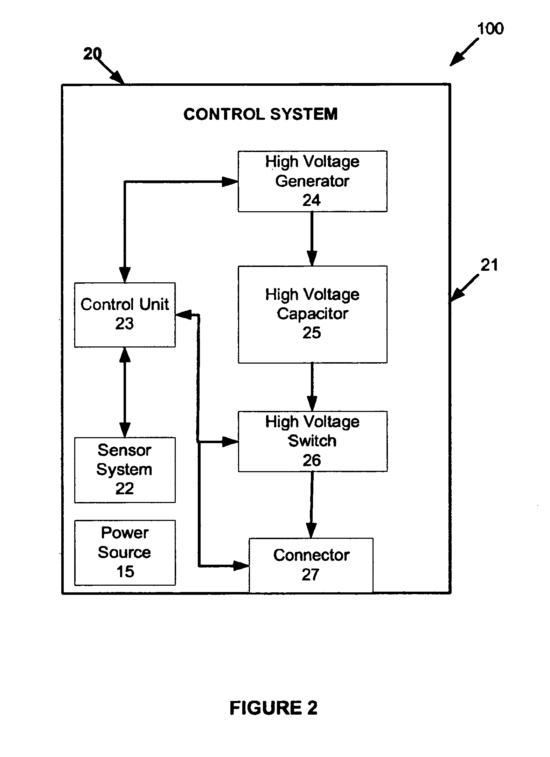

As shown in Figure 2, the control system 20 may include a defibrillator body 21, a sensor system 22, a control unit 23, a high voltage generator 24, a high voltage capacitor 25, a high voltage switch 26, a connector 27, and a power source 15. The control system 20 may include additional, different, or fewer components.

The defibrillator body 21 may be a bio-compatible housing or enclosure, canister, conductive enclosure, miniature atrial defibrillator housing, other atrial defibrillation body, or a combination thereof. The defibrillator body 21 may or may not be constructed from a conducting material. For example, all, some, or none of the defibrillator body 21 may include or be coated with metal, such as gold or titanium. The defibrillator body 21 may enclose one, some, or all of the control system 20 components. For example, the sensor system 22 may be disposed outside the defibrillator body 21 and electrically connected to the control unit 23, which is enclosed in the defibrillator body 21. In another example, as discussed below, the defibrillator body 21 may serve as an additional sensing and/or shocking electrode. Additional, different, or fewer components may be enclosed in the defibrillator body 21.

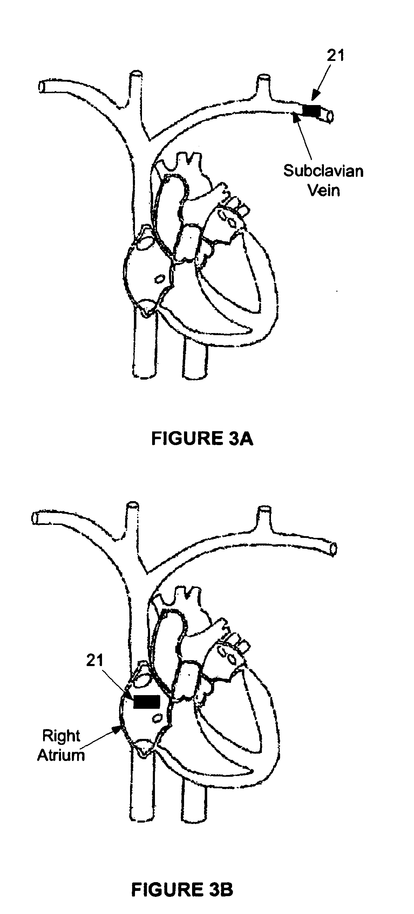

The defibrillator body 21 is sized to be implanted in the heart or surrounding regions. For example, as shown in Figure 3A, the defibrillator body 21 may be sized to be implanted into pulmonary vein, the subclavian pocket, the right atrium, or a branch of the subclavian vein. In another example, the defibrillator body 21 may be sized to be disposed outside, around, or adjacent to the pulmonary vein, the subclavian pocket, the right atrium, or a branch of the subclavian vein. In one example, as shown in Figure 3B, the defibrillator body 21 is sized to be implanted into the right atrium. The shape of defibrillator body 21 may be a box, rectangular volume, or other shaped volume that encloses system 20 components.

The size (e.g., length, height, volume) of the defibrillator body 21 depends on the size of the enclosed control system 20 components. In one embodiment, as shown in Figure 2, the defibrillator body 21 encloses at least the control unit 23, the voltage generator 24, and the high voltage capacitor 25. As discussed below, the high voltage capacitor 25 is sized to store low energy since the time duration of the electrical pulse is 30 - 100 microseconds. As used herein, the phrase "low energy" may include energy in or around the range of 0.1 - 2.0 joules, and the phrase "high energy" may include energy in or around the range of greater than 2.0 joules. A low energy capacitor may have a smaller size than a high energy capacitor. Accordingly, the defibrillator body 21 may be less than 20 cubic centimeters, and preferably, 5 - 15 cubic centimeters. The power source 15 may be a battery, power pack, or other device that provides power to one or more of the system 20 components. The power source 15 may be coupled with the sensor system 22, the control unit 23, the high voltage generator 24, the high voltage capacitor 25, the high voltage switch 26, or any combination thereof. The power source 15 provides power to the control system 20 components.

The sensor system 22 may include one or more sensing electrodes, electrocardiogram (ECG) sensors, breathing sensors, pressure sensors, acoustical sensors, blood pressure sensors, or any other sensors for sensing conditions of the heart. A condition of the heart may include muscle contraction, electrical potential, blood pressure, motion, oxygen, heart measurements, or other information relating to the operating condition of the heart, such as ECG information. ECG information may be information relating to the electrical activity of the heart over time, such as electrical potential of the heart or rhythm of the heart.

The sensor system 22 may be disposed within or outside of the defibrillator body 21.The sensor system 22may be a separate component or integrated with another component of the system 100, such as the electrode lead system 30. For example, as shown in Figure 1, the sensor system 22 may include the receiving electrode 33, which is connected to the control unit 23 (e.g., through lead 31 and the connector 27). In this example, the receiving electrode 33 functions as an electrode for delivering an electrical pulse and also as a component of the sensor system 22, for example, by providing condition signals, such as ECG signals, to the control unit 23. In another example, the sensor system 22 is connected to a pressure meter disposed in a vein, such as the vena cava, or in an atrium. Optionally, a plurality of the same or different sensors 22 may be used.

The sensor system 22 may be operable to sense a condition of a heart and emit a condition signal that identifies the condition to the control unit 23. Sensing may include receiving, detecting, determining, monitoring, or any combination thereof. The condition signal may be a cardiac functioning signal that includes information about the functioning operation of the heart. In one example, an ECG sensor may sense an electrical potential of the heart and emit a condition signal that identifies the electrical potential of the heart. The sensor system 22 may provide a condition signal to the control unit 23. The condition signal may identify the condition of the heart. The condition signal may be a processed or unprocessed signal.

The control unit 23 may process the condition signal and identify whether a state of fibrillation exists from the condition signal. Alternatively, the sensor system 22 may include a processor that identifies whether a state of fibrillation exists from the condition of the heart and emit a condition signal that indicates that an atrium is fibrillating.

In one advantageous embodiment, both the sensor 22 and the control unit 23 determine whether a state of fibrillation exists. The sensor result and the control unit result may be compared with each other. An electrical pulse may be generated when the sensor result, the control unit result, or both indicate that an atrium is fibrillating. One benefit of comparing the results is that unnecessary generation or discharge of an electrical pulse is avoided. For example, the implantable defibrillation system 100 may only generate and/or discharge an electrical pulse when both the sensor result and the control unit result indicate that a state of fibrillation exists.

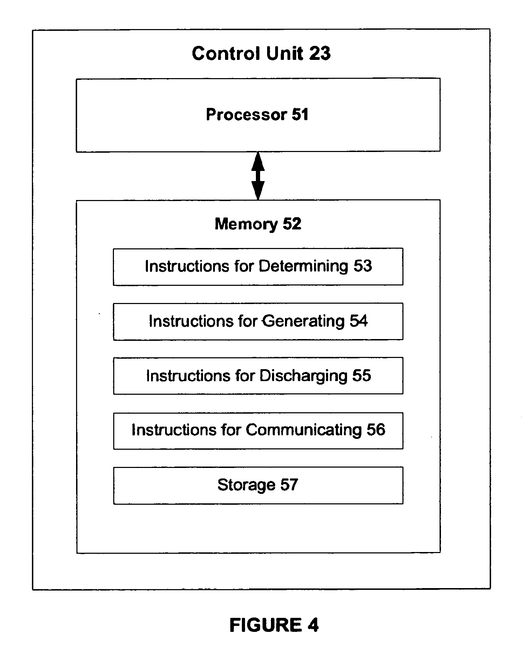

As shown in Figure 4, the control unit 23 includes a processor 51 and memory 52. The control unit 23 is a computer, processing system, or a circuit for instructing and controlling the system 20 components. The control unit 23 is coupled with the sensor system 22, the high voltage generator 24, the high voltage capacitor 25, the high voltage switch 26, the connector 27, and the power source 15. The control unit 23 controls the generation of the electrical pulse, such that the electrical pulse has a field strength of 100-700 volts/cm. Accordingly, the electrical pulse has a discharge voltage of 600 volts or greater. The control unit 23 also controls the discharge of the electrical pulses, such that one or more of the discharged electrical pulses have a time duration of 30 - 100 microseconds.

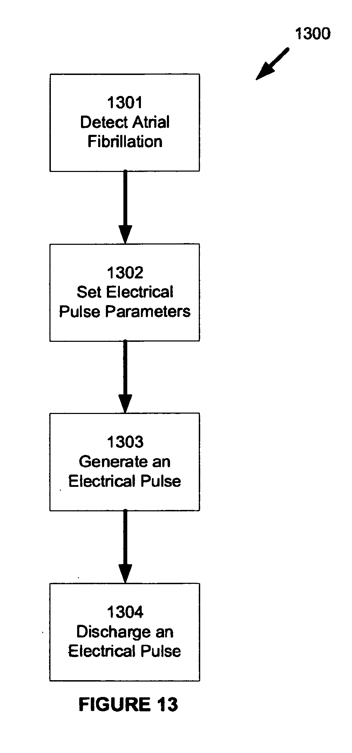

The processor 51 is a general processor, digital signal processor, application specific integrated circuit, field programmable gate array, analog circuit, digital circuit, combinations thereof, or other now known or later developed processor. The processors 23 may be a single device or a combination of devices, such as associated with a network or distributed processing. Any of various processing strategies may be used, such as multi-processing, multi-tasking, parallel processing, or the like. Processing may be local or remote. For example, a communication device may be used to transmit signals received by the processor 51 to a remote processor, which is operable to process the received signals. The processor 51 is responsive to instructions stored as part of software, hardware, integrated circuits, firmware, micro-code or the like. The processor 51 is operable to perform one or more of the acts illustrated in Figure 13.

The processor 51 is operable to determine whether a state of fibrillation exists, for example, in the atrium, based on one or more conditions of the heart. As used herein, the phrase "based on" may include as a function of, depending on, as a result of, or by analyzing. Determining whether a state of fibrillation exists may include receiving one or more condition signals from the sensor system 22. The processor 51 may analyze one or more conditions of the heart, using the information transmitted in the one or more condition signals to determine whether a state of fibrillation exists.

The processor 51 may use a fibrillation algorithm to determine whether a state of fibrillation exists. The fibrillation algorithm may use ECG information to determine whether a state of fibrillation exists. The condition signal may include ECG information. In one example, the processor 51 uses the fibrillation algorithm to analyze an electric potential of the heart over time. The processor 51 may identify a state of fibrillation based on a significant change in the ECG information.

Alternatively, or additionally, the processor 51 may map a spatial fibrillation point to determine whether a state of fibrillation exists. The spatial fibrillation point may be based on one or more conditions of the heart. The conditions may be weighted according to importance or probability that the condition indicates whether a state of fibrillation exists. The spatial fibrillation point may represent a current operating state of all or some of the heart, such as the atrium. The processor 51 may map the spatial fibrillation point on a multi-dimensional space (e.g., a graph, chart, or coordinate space). The multi-dimensional space may include a fibrillation space and a non-fibrillation space. The spatial fibrillation point may be compared to the fibrillation space and/or the non-fibrillation space. When the spatial point is in the non-fibrillation space, an atrium is not fibrillating. When the spatial point is in the fibrillation space, an atrium is fibrillating. One benefit of mapping a spatial fibrillation point is that more than one condition may be used to determine whether a state of fibrillation exists. Since multiple conditions may be used and weighted, the accuracy of determining whether a state of fibrillation exists may be increased.

The processor 51 is operable to determine electrical pulse parameters for defibrillation of an atrium. The electrical pulse parameters include a discharge voltage and an electrical pulse time duration. The electrical pulse parameters define an electrical pulse at the time of discharge from a discharge electrode 32. Once it is determined that an atrium is fibrillating, the processor 51 generates and delivers a command signal, which includes the electrical pulse parameters to one or more of the control system 20 components, such as the switch 26 or generator 24.

In one embodiment, the electrical pulse parameters define a discharge voltage of at least 600 volts. The discharge voltage is determined as a function of the field strength of the electrical pulse between the discharge electrode 32 and receiving electrode 33. For example, the discharge voltage may be determined, such that the field strength of the electrical pulse is 100-700 volts/cm. The field strength is proportional to the voltage difference between the discharge electrode 32 and the receiving electrode 33 and is inversely proportional to the distance between the discharging electrode 32 and the receiving electrode 33. Accordingly, the discharge voltage may be determined as a function of the distance from the discharge electrode 32 and the receiving electrode 33 and the desired field strength between the discharge electrode 32 and the receiving electrode 33.

The distance from the discharge electrode 32 to the receiving electrode 33 may be the shortest distance between the discharge electrode 32 and the receiving electrode 33, as placed in the patient. For example, as shown in Figure 1, the shortest distance may be distance D. Alternatively, the distance from the discharge electrode 32 to the receiving electrode 33 may be distance along the main lead 31 or sub leads 34. In one example, the distance from the discharge electrode 32 to the receiving electrode 33 may be in or around the range of 2.0 - 12.0 centimeters. Other distances may also be used.

The processor 51 is operable to activate the high voltage generator 24, which charges the high voltage capacitor 25 according to the electrical pulse parameters. The high voltage generator 24 may be activated with a command signal from the control unit 23. For example, the high voltage generator 24 may be controlled, such that the high voltage generator 24 charges the high voltage capacitor 25 to a voltage of at least 600 volts; or, more specifically, such that the electrical pulse will have a field strength of 100-700 volts/cm. In one example, the high voltage capacitor 25 may be charged to a voltage of 600 or greater volts, 1000 or greater volts, or 1300 or greater volts. In another example, the high voltage capacitor 25 may be charged to a voltage of 600 - 1000 volts. In another example, the high voltage capacitor 25 may be charged to a voltage of 1000 - 1300 volts. In another example, the high voltage capacitor 25 may be charged to a voltage of 1300 - 3000 volts.

Alternatively, or additionally, the electrical pulse may be defined to have a field strength of 100-700 volts/cm. More specifically, in one example, the electrical pulse may have a field strength of 100 - 300 volts/cm. In another example, the electrical pulse may have a field strength of 300 - 700 volts/cm. Although Figure 1 illustrates a single high voltage capacitor 25, a plurality of high voltage capacitors may be used. For example, the high voltage capacitor 25 may include a capacitor bank that is operable to provide one or more electrical pulses to the high voltage switch 26.

An electrical pulse having a field strength of 100-700 volts/cm may have a low energy. Accordingly, the size of the components may be reduced. Furthermore, an electrical pulse with a field strength of 100-700 volts/cm may ensure that the electrical pulse will defibrillate the heart, without injuring the patient's heart. A field strength of less than 100 volts/cm may be ineffective in defibrillating the heart, and a field strength of greater than 700 volts/cm may seriously injure the patient's heart.

The control unit 23 is operable to activate the high voltage switch 26. Activating the high voltage switch may include discharging energy from the high voltage capacitor 25 to a discharge electrode 32. The time duration of the electrical pulse may be controlled, such that the time duration is 30 - 100 microseconds. In one example, the time duration may be 30 - 50 microseconds. In another example, the time duration may be 50 - 70 microseconds. In another example, the time duration may be 70 - 100 microseconds.

To minimize pain, the electrical pulse has a time duration, for example, that is less than 100 microseconds. Electrical pulses that are longer than 100 microseconds may cause substantial pain and discomfort. However, in order for the electrical pulse to effectively defibrillate the heart, the time duration may be at least 30 microseconds. Electrical pulses that are shorter than 30 microseconds may require a much higher voltage to adequately defibrillate a fibrillating heart. In one range, the electrical pulse has a time duration of 30 - 100 microseconds. In this range, the electrical pulse should minimize pain and discomfort because it induces little or no skeletal muscles contraction.

The time duration ranges may be used with both the voltage ranges, as shown in Table 1, and the field strength ranges, as shown in Table 2.

**Table 1: Voltage/Duration Ranges**

| | 30-50µsec | 50-70µsec | 70-100µsec |
|---|---|---|---|
| 600 - 1000 Volts | x | x | x |
| 1000 - 1300 Volts | x | x | x |
| 1300+ Volts | x | x | x |

**Table 2: Field Strength Duration Ranges**

| | 30-50µsec | 50-70µsec | 70-100µsec |
|---|---|---|---|
| 100-300 volts/cm | x | x | x |
| 300-700 volts/cm | x | x | x |

The processor 51 is operable to control the discharge of an electrical pulse train. The electrical pulse train may include one or more electrical pulses. The electrical pulses in the electrical pulse train may have the same or different durations, discharge voltages, and/or field strength values. For example, an electrical pulse train may include a first electrical pulse and a second electrical pulse. The first electrical pulse and the second electrical pulse may have discharge voltages that are at least 600 volts and pulse durations that are 30 - 100 microseconds. In another example, the first electrical pulse has a discharge voltage that is at least 1000 volts and a pulse duration that is in the range of 30 - 100 microseconds. The second electrical pulse has a discharge voltage of less than 600 volts, a pulse duration that is less than 30 microseconds or greater than 100 microseconds, or a combination thereof.

The memory 52 is a computer readable storage media. The computer readable storage media may include various types of volatile and non-volatile storage media. The memory 52 may be a single device or a combination of devices. The memory 52 may be adjacent to, part of, networked with and/or remote from the processor 51. The memory 52 may store information, signals, or other data. As shown in Figure 4, the memory 52 may include storage 57, which is used to store information, signals, or other data. For example, the storage 57 may be used to store EKG data, monitored vital signs, patient events, log of device activity, status events, and operation parameters. Other information or data relating to or not related to atrial defibrillation may be stored in storage 57.

The memory 52 may store instructions for the processor 51. The processor 51 is programmed with and executes the instructions. The functions, acts, methods or tasks illustrated in the figures or described herein are performed by the programmed processors 51 executing instructions stored in the memory 52. The functions, acts, methods or tasks are independent of the particular type of instructions set, storage media, processor or processing strategy and may be performed by software, hardware, integrated circuits, firm ware, micro-code and the like, operating alone or in combination. The instructions are for implementing the processes, techniques, methods, or acts described herein.

As shown in Figure 4, the memory 52 may include instructions for determining fibrillation 53, instructions for generating an electrical pulse 54, instructions for discharging the electrical pulse 55, and instructions for communicating with a communication device 56. The memory 52 may include additional, different, or fewer instructions. The instructions for determining fibrillation 53 may be executed to determine whether an atrium is fibrillating. The instructions 53 may be executed to process signals, which are provided by sensors, to determine whether an atrium is fibrillating. The instructions for generating an electrical pulse 54 may be executed to generate an electrical pulse with a field strength of 100-700 volts/cm and a time duration of 30 - 100 microseconds. Accordingly, the instructions 54 may be executed to command a high voltage generator to charge a high voltage capacitor, such that an electrical pulse of at least 600 volts may be discharged from the high voltage capacitor. The instructions for discharging an electrical pulse 55 may be executed to deliver an electrical pulse having a field strength of 100-700 volts/cm and a time duration of 30 - 100 microseconds from a discharge electrode to a receiving electrode. The instructions for communicating with a communication device 56 may be executed to communicate with a communication device in, around, or outside of the defibrillation system.

The high voltage generator 24 is a generator that charges the high voltage capacitor 25. The high voltage capacitor 25 may store energy, such that an electrical pulse may be provided to a discharge electrode in accordance with the electrical pulse parameters. For example, the high voltage generator 24 may charge the high voltage capacitor 25, such that an electrical pulse, which has a voltage of at least 600 volts may be provided to the discharge electrode. In one embodiment, a shock pulse generator includes the high voltage generator 24, the high voltage capacitor 25, and the high voltage switch 26. In another embodiment, the high voltage capacitor 25 is a capacitor bank, where more than one capacitor is used to store energy, such that the energy may be selectively discharged, for example, in the pulse train option.

The high voltage switch 26 is a switch that may be activated to discharge the energy from the high voltage capacitor 25 to a discharge electrode. The high voltage switch 26 may be activated by the control unit 23 or an activation circuit. For example, the control unit 23 may activate the high voltage switch when the high voltage capacitor has been charged to the specified voltage. In another example, the high voltage switch 26 includes an activation circuit that activates the high voltage switch when one or more high voltage capacitor(s) have been charged to the specified voltage. The activation circuit may include a gas discharge tube, silicone controlled rectifier, light-activated silicon-controlled rectifier. Once activated, the high voltage switch 26 allows the high voltage capacitor 25 to discharge, thus applying a defibrillation shock to the discharge electrode. The high voltage switch 26 may reverse the high voltage polarity during the defibrillation pulse. The high voltage switch 26 may safely discharge the high voltage capacitor 25 if fibrillation stops while the high voltage capacitor 25 is charging.

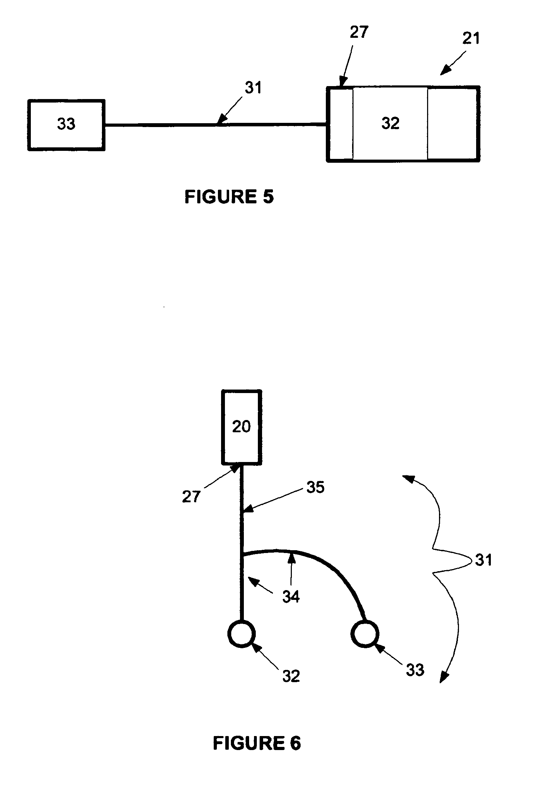

The connector 27 is a mating connector, a lead connector, or other connector for coupling the electrode lead system 30 with the control system 20. For example, as shown in Figure 2, the connector 27 is a mating connector that connects the electrode lead 31 to the high voltage switch 26 in the defibrillator body 21. In another example, as shown in Figure 5, the connector 27 couples a discharge electrode, which may be disposed in the defibrillator body 21, with a receiving electrode.

The power source 15 is a battery, power pack, or other device that provides power to one or more of the system 20 components. The power source 15 may be rechargeable. The power source 15 needed for charging the capacitor may be smaller than a power source 15 for charging a ventricular defibrillation capacitor. However, the battery drain caused by the sensing electronics module remains similar, thus the proportional size saving for the battery is smaller. Periodic battery recharging may reduce the size of the battery to substantially the size needed to produce the defibrillation shocks. In a device intended to deliver only few such shocks before being replaced or recharged, the battery size may be greatly reduced. In some embodiment, a separate battery is used for storing energy needed for defibrillation while a second battery, optionally rechargeable, powers the sensing and control electronics. The power source 15 may be inductively rechargeable, such that the power source 15 does not need to be removed from the patient to recharge.

In alternative embodiments, additional, different, or fewer components may be provided in the implantable defibrillation system 100. For example, the control system 20 may include a communication device, such as a microphone, a radio frequency (RF) device, a wireless communication device, alarm, notification device, or a combination thereof. The communication device may be used to transmit messages to another communication device, for example, positioned in a control center, such as a hospital or medical facility. The transmitted message may be fibrillation message that indicates that the patient's atrium is fibrillating and that an electrical pulse has been or will be discharged automatically. In this example, the communication device may be a radio frequency (RF) device that is used to communicate with another RF device, such as a mobile phone. Any type of message may be transmitted, for example, a phone message, a text message, a session initiation protocol message, a multimedia messaging service message, or other type of audio or data message.

In one example, the implantable defibrillation system 100 includes a microphone that is used for receiving information from an ultrasonic transducer in contact with the patient's body. In another example, the implantable defibrillation system 100 includes an alarm, such as a vibration device or acoustical device, which provides a patient with a warning before discharging the electrical pulse. The warning may be provided one or more seconds before the discharge. Accordingly, the patient may have time to prepare for the electrical pulse, for example, by pulling off to the side of the road when driving. In one embodiment, the alarm may be provided such that the patient knows to go to a hospital or medical facility and has time to make it to the hospital or medical facility. Once at the hospital or medical facility, a discharge pulse may be automatically or manually discharged, such that the defibrillation is conducted under the supervision of a medical expert. In this example, the detection and alarm are automatic; whereas, the discharge of the defibrillation electrical pulse or pulse train is manual.

In another example, the implantable defibrillation system 100 includes a positioning system, such as a location device or global positioning system. The positioning system is operable to determine a physical location of the implantable defibrillation system 100. The physical location may be transmitted to a control center, such as a hospital or medical facility. For example, a communication device may transmit a fibrillation message to a control center. The fibrillation message may include the patient's location, since the implantable defibrillation system 100 is implanted in the patient.

In another example, implantable defibrillation system 100 includes an attachment device, such as a hook device, that is coupled with a discharge electrode 32 or a receiving electrode 33 of the electrode lead system. The attachment device is operable to attach the discharge electrode or receiving electrode to a wall of the heart. Attachment may include mounting, securing, or positioning an electrode in the heart. When attached, an electrode may or may not be capable of moving relative to the heart.

The electrode lead system 30 may include a lead 31 and at least one receiving electrode 33. Additional, different, or fewer components may be provided. For example, a plurality of leads 31 and/or electrodes 32 may be provided.

As shown in Figure 5, the electrode lead 31 may be a wire, rod, flexible arm, clamp, or other device for positioning the at least one receiving electrode 33 in the heart. The lead 31 couples the discharge electrode 32 and the receiving electrode 33. The lead 31 may be electrically conductive, such that electrical signals may be transmitted between the discharge electrode 32 and the receiving electrode 33. The lead 31 may be used to transmit electrical signals between the discharge electrode 32 and the receiving electrode 33. The electrical signals may include condition signals, electrical pulses, or other communication signals.

In one embodiment, as shown in Figure 5, the control system 20 may include the discharge electrode 32. The defibrillator body 21 may enclose the discharge electrode 32. Accordingly, the connector 27 connects the discharge electrode 32 to main lead 31, such that electrical signals may be transmitted between the discharge electrode 32 and the receiving electrode 33.

In one alternative embodiment, as shown in Figure 1, the implantable defibrillation system 100 includes a communication device 60. The communication device 60 may be part of or external to the system 100. For example, the communication device 60 may be part of a different system. The communication device 60 may communicate with one or more components in the system 100, such as the control unit 23. The communication device 60 may be a radio frequency device or other wireless device. In one example, the control unit 23 may detect fibrillation and send a warning message to a radio in a hospital. The warning message may inform the medical staff at the hospital that the control system 20 detected a fibrillating atrium. In another example, the communication device 60 may be used to transmit signals to the communication device 60. For example, the control system 20 may detect a fibrillating right atrium and warn the patient that the heart is fibrillating. Once the patient arrives at the hospital, a medical attendant may use the communication device 60 to transmit a control signal to the control system 20, such that an electrical pulse is discharged from the control system 20. In this example, the medical attendant may supervise the defibrillation of the heart.

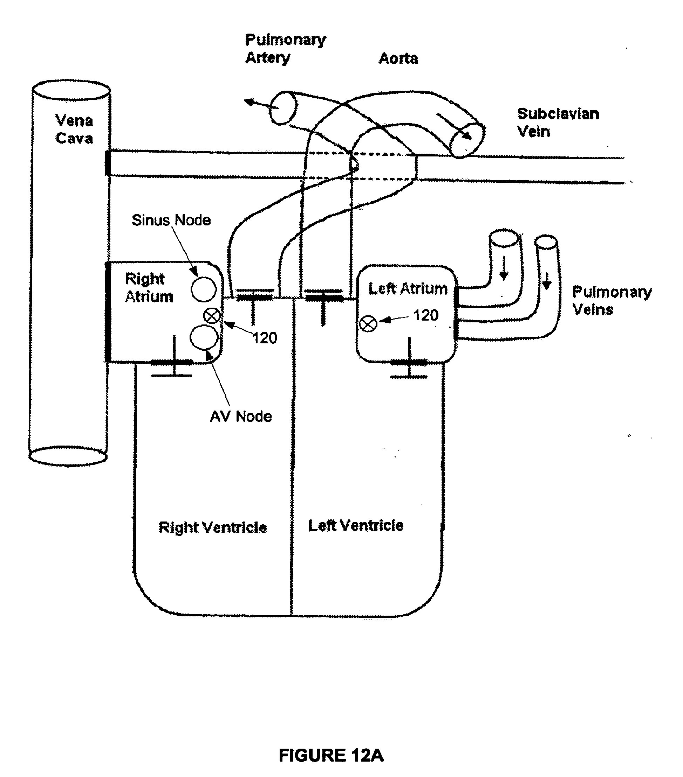

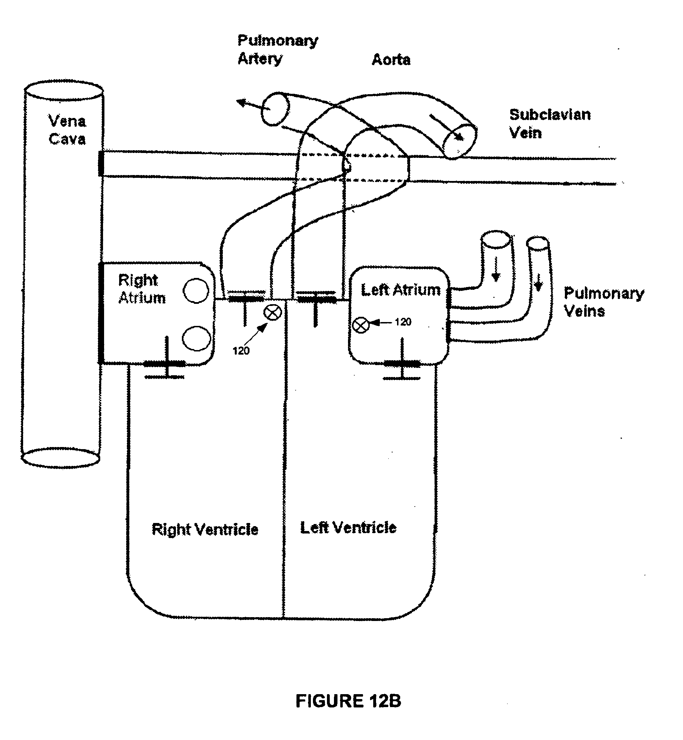

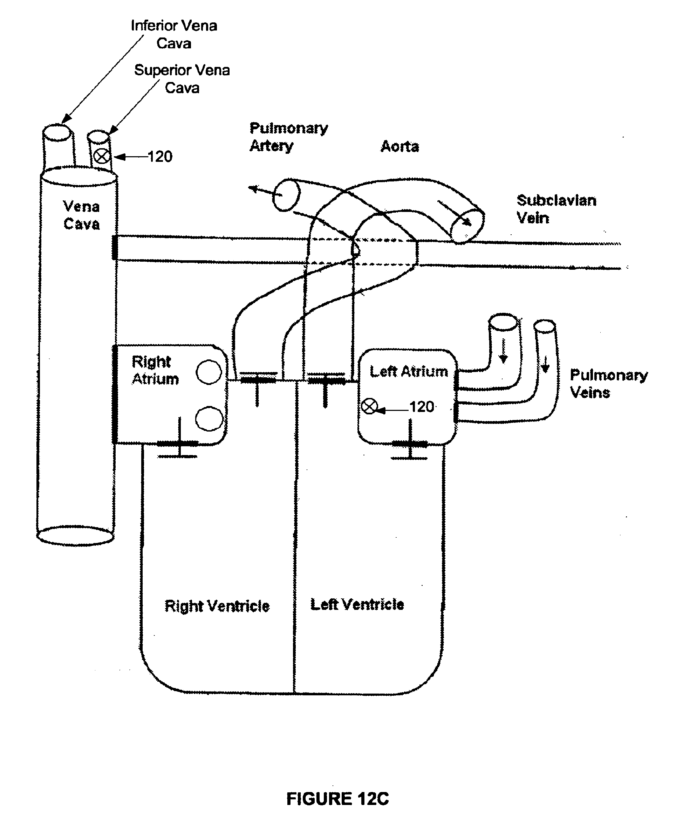

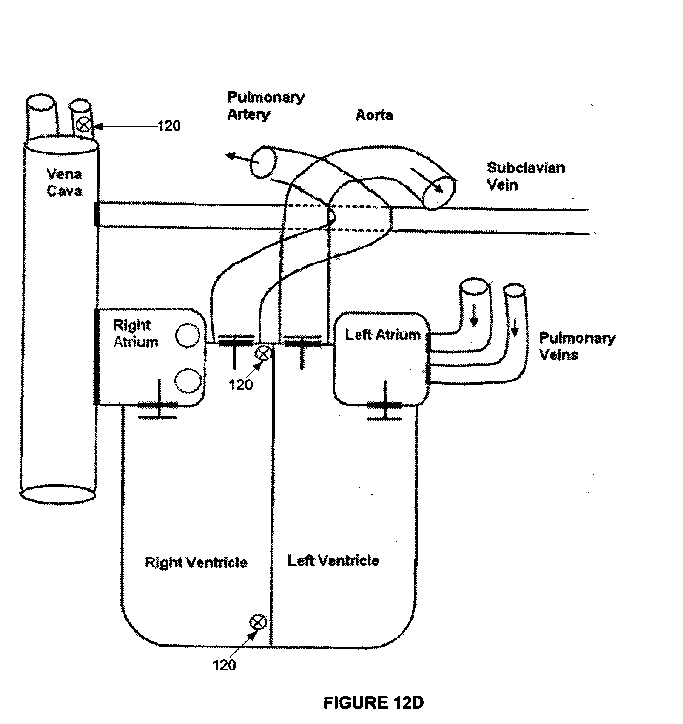

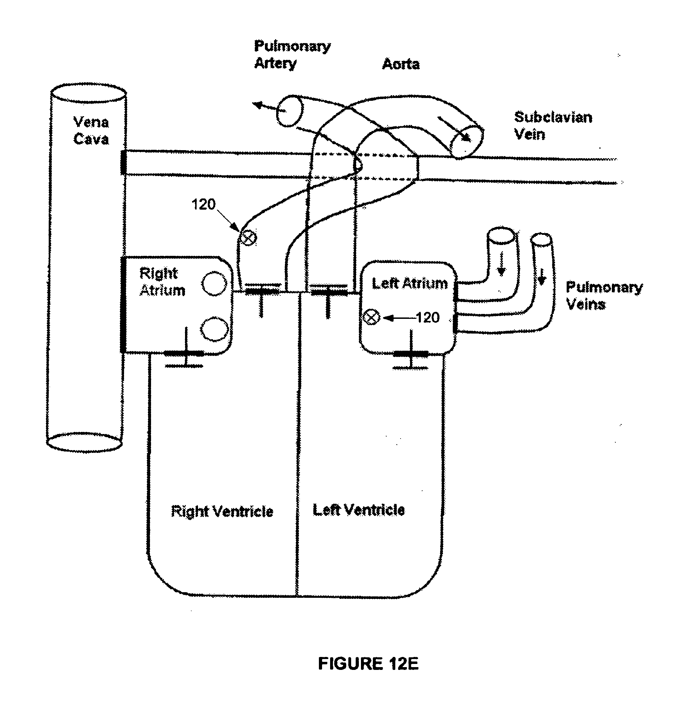

As will be discussed below, with respect to the illustrations in Figure 12, one or more electrodes may be used for sensing (e.g., with the sensing system 22), for shocking (e.g., with the high voltage generator 24, the high voltage capacitor 25, and high voltage switch 26), or a combination thereof. The electrodes and/or the electrical shocks may be disposed in the right atrium (e.g. near the AV node); the left atrium (e.g. in the coronary sinus, access: via the RA and the coronary sinus ostium); the right ventricle - near the pulmonary valve (e.g., access: via the RA and the tricuspid valve); the pulmonary artery near the pulmonary valve (e.g., access: via the RA and the tricuspid valve to the RV and then through the pulmonary valve to the pulmonary artery); the apex of the RV; or any combination thereof. With the exception of the apex of the RV, the other locations may be used for sensing and/or shocking. The right ventricular apex electrodes will be used for sensing only. In one embodiment, the defibrillator body 21 may be used as a sensing and/or shocking electrode.

In one embodiment, at least two electrodes are used for sensing and/or shocking. The at least two electrodes may be any combination of shocking electrodes and sensing electrodes. Shocking electrodes may be located in the right atrium; the left atrium; the right ventricle (e.g., near the pulmonary valve); or the pulmonary artery near the pulmonary valve. Sensing electrodes may be located in the right atrium; the left atrium; the right ventricle (e.g., near the pulmonary valve); the pulmonary artery near the pulmonary valve; the apex of the RV; or the defibrillator body 21.

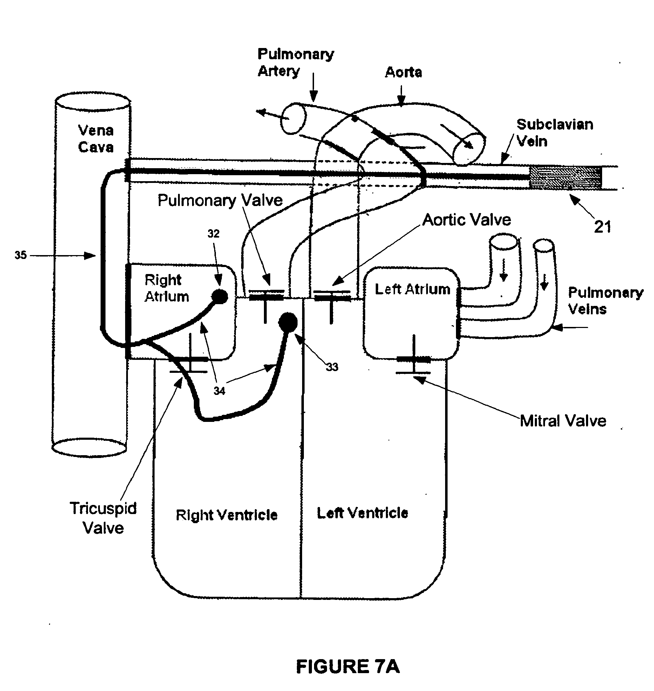

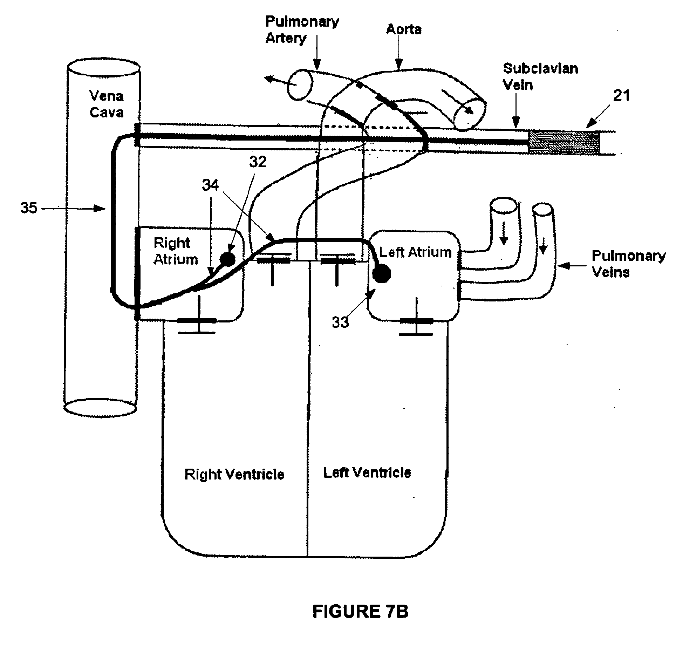

Figures 6, 8, and 10 illustrate examples of different embodiments of the implantable defibrillation system 100. Figures 7, 9, and 11 illustrate different locations for the electrodes in the implantable defibrillation system 100. The examples shown in Figures 6 - 11 are not limiting. Other embodiments of the implantable defibrillation system 100 and/or other locations of the electrodes may be used for atrial defibrillation.

Figure 6 illustrates an electrode lead system 30 with a bifurcated lead. The electrode lead 31 includes a main lead 35 and sub leads 34. An electrode, a sensor, or a combination thereof may be disposed at one end of the sub leads 34. The other ends of the sub leads 34 may be coupled with the main lead 35. For example, a first sub lead may include a first receiving electrode at one end of the first sub lead, and a second sub lead may include a second receiving electrode at one end of the second sub lead. The other ends of the first and second sub leads may be connected to the main lead 35. The lengths of the main lead 35 and one or more sub leads 34 may be the same or different. For example, a first sub lead may be longer than a second sub lead.

Figures 7A and 7B illustrate electrode lead systems 30 with bifurcated leads, as positioned in the patient. Figure 7A illustrates a bifurcated lead that is located in the subclavian vein, however, may be placed in the vena cava. The main lead 35 traverses the subclavian vein and enters the vena cava. The two sub leads 34 enter the Right Atrium (RA) and the Right Ventricle (RV), respectively. The longer sub-lead 34 enters the RV through the tricuspid valve. The distal ends of the sub leads. 34 may be anchored to walls of the Right Atrium (RA) (in various locations including the AV node or the SA node) and the Right Ventricle (RV) such that the shock delivering electrodes are in contact to the walls of the right atrium and the right ventricle. Figure 7B illustrates a bifurcated lead as positioned in the patient. The defibrillator body 21 may be located in a side branch of the subclavian vein. The main lead 35 enters the heart through the vena cava. A first sub-lead 34, having a distal first electrode 32, is located in the Right Atrium (RA). The discharge electrode 32 may be anchored to a RA wall, preferably near the pulmonary valve. A second, preferably longer sub-lead 34 is inserted via the coronary sinus. The second sub-lead 34 comprises a receiving electrode 33 which is located in the Left Atrium (LA) wall near the pulmonary valve.

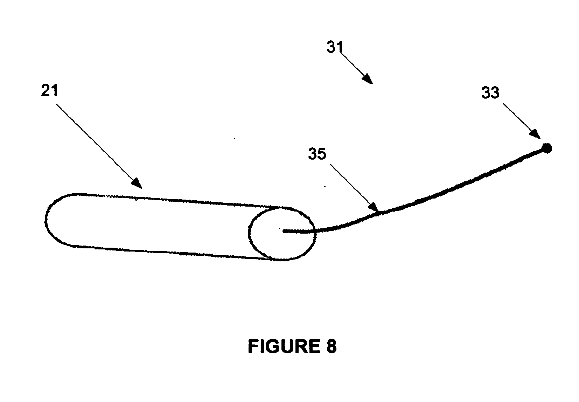

Figure 8 illustrates an electrode lead system 30 with a receiving electrode 33 disposed at one end of the electrode lead 31. The electrode lead 31 includes a main lead 35. An electrode, a sensor, or a combination thereof is disposed on the main lead 35. The main lead 35 may be coupled with the control system 20. The discharge electrode 32 may be disposed in the defibrillator body 21, for example, as shown in Figure 5. In one embodiment, the defibrillator body 21 may be connected to the lead system 31 via a special connector. The defibrillator body 21 may be displaced from the leads, for example, subcutaneously implanted, like current pacemakers and defibrillators.

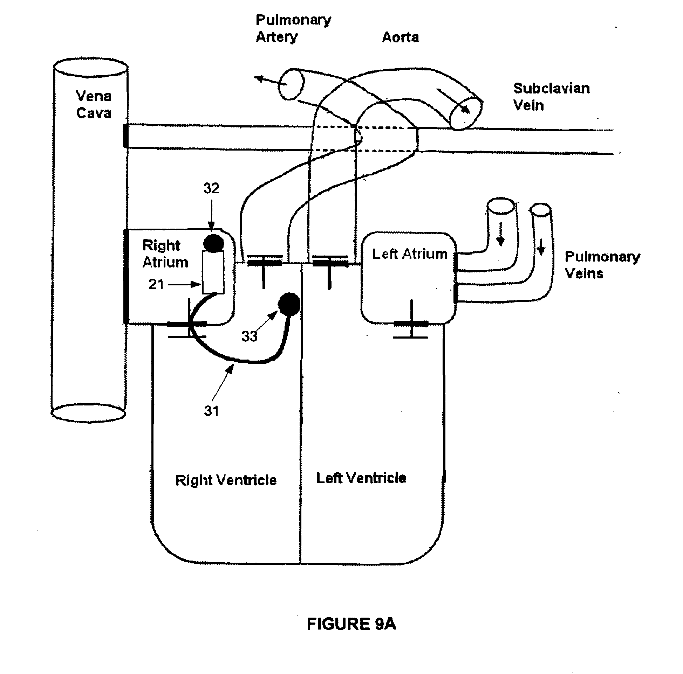

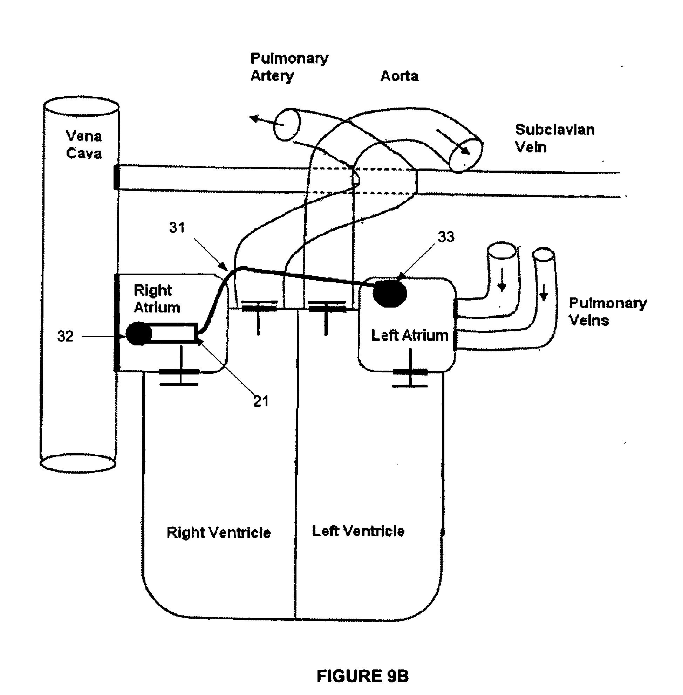

Figures 9A and 9B illustrate electrode lead systems 30 with a discharge electrode 32 disposed at one end of the electrode lead 31, as positioned in the patient. Figure 9A illustrates an electrode lead system 30 with a receiving electrode 33 disposed at one end of the electrode lead 31. The defibrillator body 21, acting as, or including a discharge electrode 32 may be located in the Right Atrium (RA). The defibrillator body 21 may be anchored to the RA wall. Accordingly, the shock delivering electrode, such as the discharge electrode 32, is in contact to the RA wall. A single lead having a receiving electrode 33 enters the Right Ventricle (RV) through the Tricuspid valve. The receiving electrode 33 may be anchored to wall of the RV, preferably such that discharge electrode 33 is in contact to the RV wall. Figure 9B illustrates a defibrillator body 21, acting as, or comprising a discharge electrode 32. The defibrillator body 21 and/or the discharge electrode 32 may be located in the RA. The defibrillator body 21 is anchored to the RA wall. The discharge electrode 21 is in contact to the RA wall. A single lead 31 includes a receiving electrode 33 that is inserted into the Coronary Sinus or one of the veins leading to it.

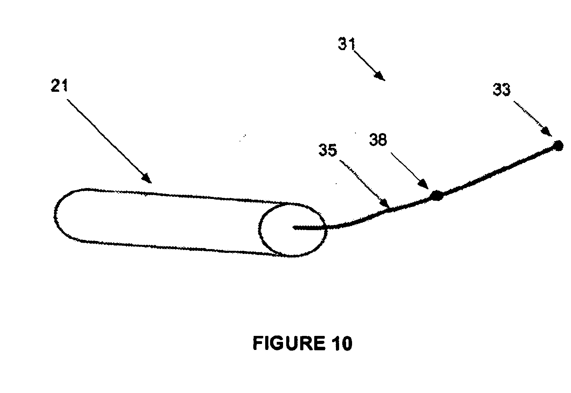

Figure 10 illustrates an electrode lead system 30 with a pigtail lead. A plurality of electrodes, sensors, or a combination thereof is disposed on the main lead 31. For example, a central electrode 38 may be disposed on the main lead 31 between the receiving electrode 33 and the control system 20. The central electrode 38 may be a sensing electrode (e.g., part of the sensing system 22) or a discharge electrode 32 for discharging the electrical pulse.

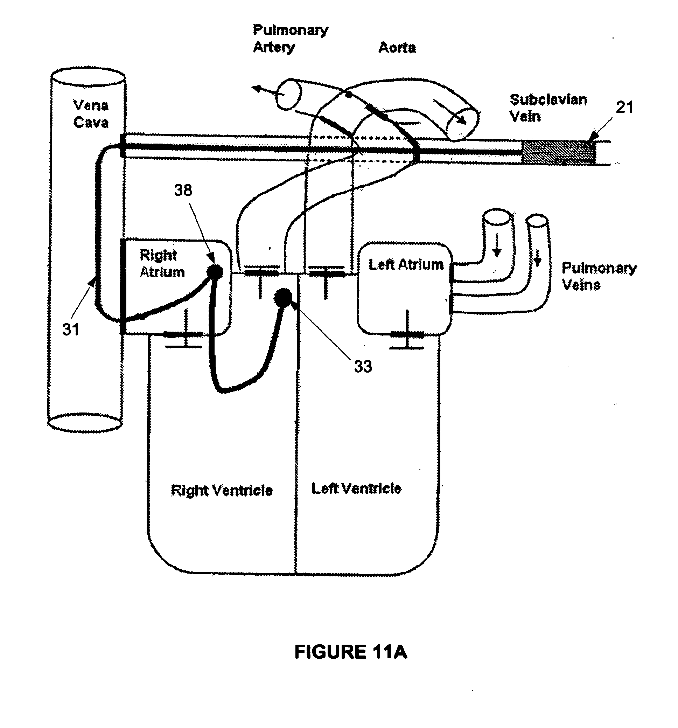

Figure 11A illustrates more than one electrode on the main lead 31. The defibrillator body 21 may be located in a side branch of the subclavian vein. The main lead 31 includes at least a central electrode 38 and a receiving electrode 33. The main lead 31 enters the heart through the vena cava. The central electrode 38 is positioned in the Right Atrium (RA). The distal section of the main lead 31 is inserted via the tricuspid valve into the RV. The receiving electrode 33, which is located in the Right Ventricle (RV), may be anchored to the RV wall near the pulmonary valve.

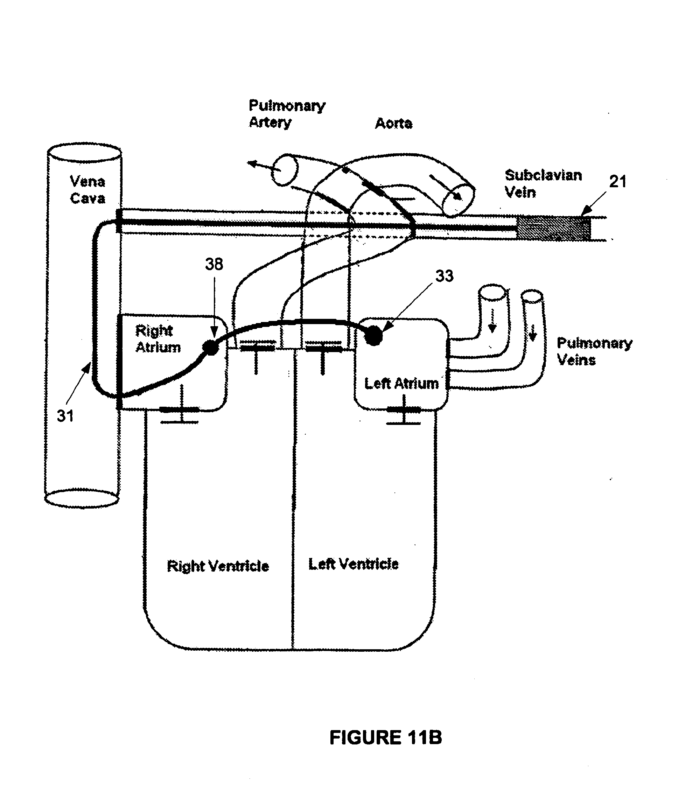

Figure 11B also illustrates more than one electrode on the main lead 31 as positioned in the patient. The defibrillator body 21 is located in the subclavian vein. The main lead 31 includes a central electrode 38 and a receiving electrode 33. The main lead 31 enters the heart through the vena cava. The central electrode is located in the Right Atrium (RA). Optionally, the central electrode 38 may be anchored to the RA wall, preferably near the pulmonary valve. The receiving electrode 33 of the main lead 31 is inserted into the coronary sinus or one of the veins leading to it.

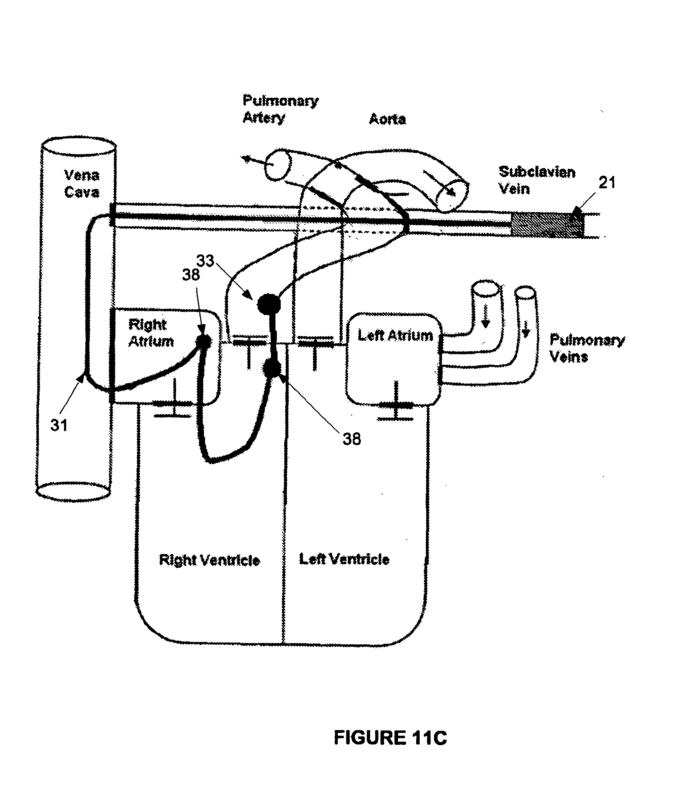

Figure 11C also illustrates more than one electrode on the main lead 31 as positioned in the patient. The defibrillator body 21 may be located in a side branch of the subclavian vein. The main lead 31 includes at least two, and optionally three electrodes. The main lead 31 enters the heart through the vena cava. The first central electrode 38, which may be a sensing electrode or discharging electrode, is positioned in the Right Atrium (RA). A central section of the main lead 31 is inserted via the tricuspid valve into the RV. The second central electrode 38, which is located in the Right Ventricle (RV), may be anchored to the RV wall near the pulmonary valve. The receiving section of the main lead 31 is inserted via the pulmonary valve into the pulmonary artery. The receiving electrode 33 is located in the pulmonary artery.

The discharge electrode 32 and receiving electrode 33 may be disposed in the heart. Figures 12A - 12E show examples of different locations of the various electrodes, such as the sensing electrode, discharge electrode 32 and receiving electrode 33. As used in Figure 12, the electrode 120 may be a sensing electrode, discharge electrode 32, receiving electrode 33, central electrode 39, or any combination thereof. The position of the electrode 120 in Figures 12A - 12E may the physical location of the electrode or the physical location of the electrical shock provided by the electrode. For example, the actual position of the electrode 120 may be provided in the Coronary Sinus (or one of the veins surrounding the Left Atrium) and provide an electrical shock to the left atrium. In Figures 12A - 12E, the electrical shock to the left atrium may be illustrated as the position of the electrode 120, even though the actual electrode may be disposed away from or adjacent to the position of the electrode 120 in the illustrations.

Figure 12A shows a first electrode 120 that is located near the antrioventricular (AV) node inside the RA. The first electrode 120 may be inserted via the vena cava. A second electrode 120 is located at the wall of the LA (or intra-atrial septum) near the pulmonary valve. The second electrode 120 is preferably inserted via the coronary sinus.

Figure 12B shows a first electrode 120 located at the RV near pulmonary valve. The first electrode 120 may be inserted via the vena cava and the RV valve. A second electrode 120 is located at the wall of the left atrium (LA) (or intra-atrial septum) near the pulmonary valve. Preferably, the second electrode 120 may be inserted via the coronary sinus.

Figure 12C shows a first electrode 120 located at the superior vena cava. A second electrode 120 is located at the wall of the LA (or intra-atrial septum) near the pulmonary valve. The second electrode 120 may be inserted via the coronary sinus.

As shown in Figure 12D, a first electrode 120 may be located at the superior vena cava and a second electrode 120 is located in the RV near the pulmonary valve. The second electrode 120 may be inserted via the tricuspid valve. A third electrode 120 at the RV apex may be inserted via the tricuspid valve and used for sensing and/or ventricular defibrillation and/or pacing.

As shown in Figure 12E, a first electrode 120 may be located in the pulmonary artery, near the pulmonary valve. The first electrode may be provided in the pulmonary artery via the right atrium and the tricuspid valve to the right ventricle. Once in the right ventricle, the first electrode 120 may be moved through the pulmonary valve to the pulmonary artery. A second electrode 120 may be located in the Coronary Sinus or one of the veins leading to it.

In one advantageous embodiment, the electrode lead system 30 includes one or more additional electrodes that are operable to deliver cardiac pacing pulses and/or ventricular defibrillation. One benefit of using additional electrodes that are operable to deliver cardiac pacing pulses and/or ventricular defibrillation is that the defibrillation system 100 may be used when atrial fibrillation progresses to ventricular fibrillation or ventricular arrhythmia or when the delivered atrial defibrillation shock induces ventricular fibrillation or ventricular arrhythmia. The additional electrodes may also be used such that the delivered atrial defibrillation shock is synchronized to the natural or paced ventricular beat. One benefit of synchronizing the shock and the natural or paced ventricular beat is that the probability that the delivered atrial defibrillation shock would cause ventricular fibrillation or ventricular arrhythmia is reduced.

In one embodiment, the implantable defibrillation system 100 includes a drug delivering system. The drug delivery system may include a drug pump that is capable of injecting a drug into a right atrium. The drug pump may be computer controlled or manually controlled. For example, the drug pump may be controlled by the control unit 23. The control unit 23 may activate the drug pump prior to the discharge of the electrical pulse. The drug delivery may further reduce the pain and/or discomfort of atrial defibrillation. Furthermore, delivering a drug directly into the heart may act quicker than delivering the drug using intravenous (IV) therapy because drugs that are provided using IV therapy may need to travel to the heart. Accordingly, the travel time may slow down the defibrillation process.

Figure 13 is a flow diagram illustrating a method 1300 for defibrillating an atrium with an implantable defibrillation system. The method 1300 includes detecting when an atrium of a heart fibrillates 1301, setting electrical pulse parameters 1302, generating an electrical pulse 1303, and discharging an electrical pulse 1304. The method 1300 may include additional, different, or fewer acts.

In act 1301, an implantable defibrillation system detects when an atrium of a heart fibrillates. Detecting fibrillation may include receiving sensor signals, such as condition signals, from one or more sensors. The sensor signals may be processed to determine whether an atrium is in a fibrillation state. Processing the sensor signals may include signal processing, spatial comparisons, or other processes of determining whether a sensor signal indicates that an atrium is fibrillating.

In act 1302, electrical pulse parameters are set prior to or upon detection of atrial defibrillation. The electrical pulse parameters define characteristics, values, boundaries, or limitations of the electrical pulse. For example, the electrical pulse parameters may define a discharge voltage and a time duration of one or more electrical pulses. The electrical pulse parameters may be determined as a function of a distance between a discharge electrode and a receiving electrode and/or voltage difference between a discharge electrode and a receiving electrode.

The electrical pulse parameters may define an electrical pulse with a field strength of 100 - 700 volts/cm. Accordingly, the electrical pulse may have a defibrillation voltage of at least 600 volts. In act 1303, the implantable defibrillation system generates an electrical pulse in accordance with the electrical pulse parameters. Generating an electrical pulse may include charging a high voltage capacitor with energy, such that an electrical pulse in accordance with the electrical pulse parameters may be discharged from the high voltage capacitor. In act 1304, the implantable defibrillation system discharges the electrical pulse to an atrium of the heart using a discharge electrode and a receiving electrode. Discharging the electrical pulse may include providing energy from a high voltage capacitor to a discharge electrode. Discharging the electrical pulse may also include controlling the discharge, such that a time duration of the electrical pulse is 30 - 100 microseconds.

The method 1300 may further include discharging an electrical pulse train that includes the first electrical pulse and a second electrical pulse. The first electrical pulse and a second electrical pulse may have the same or different discharge voltage, time duration, field strength, or a combination thereof. In one embodiment, two or more pulses may be included in the pulse train. The electrical pulses of the electrical pulse train may be monophasic or biphasic. The pulse train may include electrical pulses that have or do not have the same polarity as the other electrical pulses in the electrical pulse train.

The method 1300 may include other acts. For example, the method 1300 may include implanting the implantable defibrillation system into a heart. The implantable defibrillation system may be implanted such that a distance between the discharge electrode and the receiving electrode is less than 3 centimeters.

In another example, the method 1300 includes notification acts. The method 1300 may be used to notify or communicate with a patient or a control center, such as a hospital or a medical control center. To notify a patient, a notification system may be activated. The notification is operable to notify a patient of the first electrical pulse before discharging the first electrical pulse to the atrium of the heart. Notifying the patient may include activating a vibration device or acoustic device. To notify a control center, a message may be transmitted to a communication device. The message may be fibrillation message that indicates that an atrium is fibrillating and an electrical pulse has been or will be discharged. Other message may be transmitted to the communication device, such as a location message that indicates the location of the implantable defibrillation system. The location may be determined using a positioning system, such as a location device or global positioning system.

The method 1300 may also include pain reduction acts. The pain reduction acts may include delivering a drug to the heart using the implantable defibrillation system before discharging the first electrical pulse to the atrium of the heart. Other pain reduction acts may be provided.

Various embodiments described herein can be used alone or in combination with one another. The forgoing detailed description has described only a few of the many possible implementations of the present invention. For this reason, this detailed description is intended by way of illustration, and not by way of limitation. It is only the following claims, including all equivalents that are intended to define the scope of this invention.

## Claims

1. An implantable heart defibrillator (100) for use with an electrode lead system (30), the implantable heart defibrillator comprising:
an electrode lead connector (27) connectable to the electrode lead system,
a sensor (22) connected to the electrode lead connector, the sensor being operable to sense a condition of a heart and emit a condition signal that identifies the condition;
a control unit (23) connected to the sensor, the control unit being operable to identify whether a state of atrial fibrillation exists from the condition signal and emit a command signal if the state of atrial fibrillation exists; and
and a shock pulse generator (24) connected to the control unit and the electrode lead connector, the shock pulse generator being operable to emit at least one defibrillation shock to right and left atria of the heart via the electrode lead connector upon receipt of the command signal, wherein the at least one defibrillation shock comprises at least one pulse having a voltage greater than or equal to 600 volts , **characterized in that** the at least one pulse has a time duration of 30 to 100 microseconds, and that the control unit cooperates with the shock pulse generator to synchronize the defibrillation shock with the electrocardiogram cycle of the heart.

2. The implantable heart defibrillator of claim 1, wherein the at least one pulse has a field strength of 100 to 700 volts/cm.

3. The implantable heart defibrillator of claim 1, wherein the voltage of the at least one pulse is determined as a function of a distance between at least two electrodes of the electrode lead system.

4. The implantable heart defibrillator of claim 1, wherein the at least one pulse is a monophasic pulse or a biphasic pulse.

5. The implantable heart defibrillator of claim 1, further comprising wireless communication circuitry in communication with the control unit configured to receive a defibrillation command to remotely trigger the at least one defibrillation shock.

6. The implantable heart defibrillator of claim 1, wherein the value of the voltage and time duration of the at least one pulse are configured to reduce pain caused by the defibrillation shock.

7. A system (100) for defibrillation of an atrium of a heart, the system comprising:
a memory (52), and
a processor (51) in communication with the memory, the memory including processor readable instructions executable with the processor, wherein the processor readable instructions are executable to:
receive condition signals from one or more sensors, the condition signals identifying a condition of a heart;
determine when the atrium of the heart is in a fibrillation state using the condition signals;
generate a first defibrillation electrical pulse with a field strength of 100 to 700 volts/cm between a discharge electrode (32) and a receiving (33) electrode positioned in right and left atria of the heart and a time duration of 30 to 100 microseconds; and
discharge the first defibrillation electrical pulse from the discharge electrode and the receiving electrode to defibrillate the atrium of the heart, wherein the discharge of the first defibrillation electrical pulse is synchronized with the electrocardiogram cycle of the heart.

8. The system of claim 7, wherein the first defibrillation electrical pulse has a discharge voltage of at least 600 volts.

9. The system of claim 7, wherein the processor readable instructions are further executable to receive a defibrillation command from outside of a patient for remote triggering of the first defibrillation electrical pulse.

## Patentansprüche

1. Implantierbarer Defibrillator (100) zur Verwendung mit einem Elektrodenanschlusssystem (30), wobei der implantierbare Defibrillator Folgendes aufweist:
einen Elektrodenanschlussverbinder (27), der mit dem Elektrodenanschlusssystem verbindbar ist;
einen Sensor (22), der mit dem Elektrodenanschlussverbinder verbunden ist, wobei der Sensor zum Erfassen eines Herzzustands und zum Ausgeben eines den Zustand identifizierenden Zustandssignals betrieben werden kann;
eine Steuereinheit (23), die mit dem Sensor verbunden ist, wobei die Steuereinheit betrieben werden kann, um aus dem Zustandssignal einen Zustand eines Vorliegens von Vorhofflimmern zu identifizieren und ein Anweisungssignal auszugeben, wenn der Zustand eines Vorhofflimmerns vorliegt; und
einen Schockimpulsgenerator (24), der mit der Steuereinheit und dem Elektrodenanschlussverbinder verbunden ist, wobei der Schockimpulsgenerator betrieben werden kann, um bei Erhalt des Anweisungssignals über den Elektrodenanschlussverbinder zumindest einen Defibrillationsschock an den rechten und den linken Vorhof des Herzens abzugeben, wobei der zumindest eine Defibrillationsschock zumindest einen Impuls mit einer Spannung von 600 Volt oder darüber aufweist,
**dadurch gekennzeichnet, dass**
der zumindest eine Impuls eine Zeitdauer von 30 bis 100 Mikrosekunden aufweist, und dass die Steuereinheit mit dem Schockimpulsgenerator zur Synchronisation des Defibrillationsschocks mit dem Elektrokardiogrammrhytmus des Herzens zusammenwirkt.

2. Implantierbarer Defibrillator nach Anspruch 1, worin der zumindest eine Impuls eine Feldstärke von 100 bis 700 Volt/ cm aufweist.

3. Implantierbarer Defibrillator nach Anspruch 1, worin die Spannung des zumindest einen Impulses als Funktion eines Abstandes zwischen zumindest zwei Elektroden des Elektrodenanschlusssystems bestimmt wird.

4. Implantierbarer Defibrillator nach Anspruch 1, worin es sich bei dem zumindest einen Impuls um einen monophasischen Impuls oder einen biphasischen Impuls handelt.

5. Implantierbarer Defibrillator nach Anspruch 1, der ferner einen mit der Steuereinheit in Verbindung stehenden Funkübertragungsschaltkreis aufweist, der zum Empfangen einer Defibrillationsanweisung ausgebildet ist, um den zumindest einen Defibrillationsschock ferngesteuert auszulösen.

6. Implantierbarer Defibrillator nach Anspruch 1, worin Spannungswert und Zeitdauer des zumindest einen Impulses zum Verringern der durch den Defibrillationsschock verursachten Schmerzen ausgebildet sind.

7. System (100) zum Defibrillieren eines Vorhofes eines Herzens, wobei das System Folgendes aufweist:
einen Speicher (52); und
eine Datenverarbeitungseinrichtung (51), die mit dem Speicher in Verbindung steht, wobei der Speicher für eine Datenverarbeitungseinrichtung lesbare Anweisungen enthält, die von der Datenverarbeitungseinrichtung ausgeführt werden können, und wobei die für eine Datenverarbeitungseinrichtung lesbaren Anweisungen ausgeführt werden können, um:
von einem oder mehreren Sensoren Zustandssignale zu erhalten, wobei die Zustandssignale einen Herzzustand identifizieren;
unter Verwendung des Zustandssignals festzustellen, wann sich der Vorhof des Herzens in einem Flimmerzustand befindet;
zwischen einer am rechten Vorhof des Herzens positionierten Abgabeelektrode (32) und einer am linken Vorhof des Herzens positionierten Aufnahmeelektrode (33) einen ersten elektrischen Defibrillationsimpuls mit einer Feldstärke von 100 bis 700 Volt/cm und einer Zeitdauer von 30 bis 100 Mikrosekunden zu erzeugen; und
Abgeben des ersten elektrischen Defibrillationsimpulses über die Abgabeelektrode und die Aufnahmeelektrode zum Defibrillieren des Vorhofes des Herzens, wobei die Abgabe des ersten elektrischen Defibrillationsimpulses mit dem Elektrokardiogrammrhythmus des Herzens synchronisiert wird.

8. System nach Anspruch 7, worin der erste elektrische Defibrillationsimpuls eine Entladungsspannung von wenigstens 600 Volt aufweist.

9. System nach Anspruch 7, worin die für eine Datenverarbeitungseinrichtung lesbaren Anweisungen ferner ausführbar sind, um von außerhalb eines Patienten eine Defibrillationsanweisung zum ferngesteuerten Auslösen des ersten elektrischen Defibrillationsimpulses zu erhalten.

## Revendications

1. Défibrillateur cardiaque implantable (100) pour utilisation avec un système de fil d'électrode (30), le défibrillateur cardiaque implantable comprenant :
un connecteur de fil d'électrode (27) apte à être connecté au système de fil d'électrode ;
un capteur (22) connecté au connecteur de fil d'électrode, le capteur étant apte à détecter un état d'un coeur et à émettre un signal d'état qui identifie l'état ;
une unité de commande (23) connectée au capteur, l'unité de commande étant apte à identifier si un état de fibrillation atriale existe à partir du signal d'état et émet un signal de commande si l'état de fibrillation atriale existe, et
un générateur d'impulsions de choc (24) connecté à l'unité de commande et au connecteur de fil d'électrode, le générateur d'impulsions de choc étant apte à émettre au moins un choc de défibrillation à l'oreillette droite et gauche du coeur par le connecteur de fil d'électrode lors de la réception du signal de commande, où le au moins un choc de défibrillation comprend au moins une impulsion ayant une tension supérieure ou égale à 600 volts, **caractérisé en ce que** la au moins une impulsion a une durée de temps de 30 à 100 microsecondes, et que l'unité de commande coopère avec le générateur d'impulsions de choc afin de synchroniser le choc de défibrillation avec le cycle d'électrocardiogramme du coeur.

2. Défibrillateur cardiaque implantable selon la revendication 1, dans lequel la au moins une impulsion possède une intensité de champ de 100 à 700 volts/cm.

3. Défibrillateur cardiaque implantable selon la revendication 1, dans lequel la tension de la au moins une impulsion est déterminée comme une fonction d'une distance entre au moins deux électrodes du système de fil d'électrode.

4. Défibrillateur cardiaque implantable selon la revendication 1, dans lequel la au moins une impulsion est une impulsion monophasique ou une impulsion biphasique.

5. Défibrillateur cardiaque implantable selon la revendication 1, comprenant en outre un circuit de communication sans fil en communication avec l'unité de commande configurée pour recevoir une instruction de défibrillation afin de déclencher à distance le au moins un choc de défibrillation.

6. Défibrillateur cardiaque implantable selon la revendication 1, dans lequel la valeur de la tension et la durée de temps de la au moins une impulsion sont configurées pour réduire la douleur provoquée par le choc de défibrillation.

7. Système (100) pour la défibrillation d'une oreillette d'un coeur, le système comprenant :
une mémoire (52) ; et
un processeur (51) en communication avec la mémoire, la mémoire incluant des instructions lisibles par le processeur aptes à être exécutées avec le processeur, où les instructions lisibles par le processeur sont exécutables pour :
recevoir des signaux d'état d'un ou de plusieurs capteurs, les signaux de condition identifiant une condition d'un coeur ;
déterminer lorsque l'oreillette du coeur est dans un état de fibrillation en utilisant les signaux d'état ;
produire une première impulsion électrique de défibrillation avec une intensité de champ de 100 à 700 volts/cm entre une électrode de décharge (32) et une électrode de réception (33) positionnée dans les oreillettes droite et gauche du coeur et une durée de temps de 30 à 100 microsecondes ; et
décharger la première impulsion électrique de défibrillation de l'électrode de décharge et de l'électrode de réception pour la défibrillation de l'oreillette du coeur, où la décharge de la première impulsion électrique de défibrillation est synchronisée avec le cycle de l'électrocardiogramme du coeur.

8. Système selon la revendication 7, dans lequel la première impulsion électrique de défibrillation a une tension de décharge d'au moins 600 volts.

9. Système selon la revendication 7, dans lequel les instructions lisibles par le processeur sont en outre exécutables pour recevoir une instruction de défibrillation depuis l'extérieur d'un patient afin de déclencher à distance la première impulsion électrique de défibrillation.
